Europäisches Patentamt

European Patent Office

Office européen des brevets

(19)

(11) Veröffentlichungsnummer : **0 134 227**
**B1**

(12) **EUROPÄISCHE PATENTSCHRIFT**

(45) Veröffentlichungstag der Patentschrift :
28.05.86

(21) Anmeldenummer : 84900610.1

(22) Anmeldetag : 26.01.84

(86) Internationale Anmeldenummer :
PCT/DE 84/00021

(87) Internationale Veröffentlichungsnummer :
WO/8402840 (02.08.84 Gazette 84/18)

(51) Int. Cl.⁴ : **A 61 F 13/20**

(54) TAMPONPACKUNG.

(30) Priorität : 28.01.83 DE 3302932

(43) Veröffentlichungstag der Anmeldung :
20.03.85 Patentblatt 85/12

(45) Bekanntmachung des Hinweises auf die Patenterteilung : 28.05.86 Patentblatt 86/22

(84) Benannte Vertragsstaaten :
FR GB

(56) Entgegenhaltungen :
DE-A- 1 909 578
FR-A-   964 603
US-A- 2 413 480
US-A- 3 139 886
US-A- 3 674 025

(73) Patentinhaber : JOHNSON & JOHNSON GmbH
Kaiserswerther Strasse 270
D-4000 Düsseldorf 30 (DE)

(72) Erfinder : FRIESE, Axel
Weststrasse 68
D-5600 Wuppertal-Elberfeld (DE)

(74) Vertreter : Groening, Hans Wilhelm, Dipl.-Ing.
Siebertstrasse 4 Postfach 860 340
D-8000 München 86 (DE)

**Beschreibung**

Die Erfindung betrifft eine Tamponpackung, insbesondere für die Frauenhygiene, bestehend aus einem Tamponapplikator, der eine Applikatorhülse und einen darin teleskopartig axial bewegbar gelagerten Schieber aufweist, sowie aus einem in der Applikatorhülse angeordneten Tampon mit Rückholband, der mittels des Schiebers aus der Applikatorhülse ausstoßbar ist, wobei eine flexible Verpackungsfolie im Bereich des Einführendes des Tampons angeordnet und an der Applikatorhülse gehalten ist.

Derartige Tamponpackungen sind bekannt. Beispielsweise ist in der GB-A-1 262 887 ein Tamponapplikator beschrieben, der im wesentlichen aus einer Applikatorhülse und einem darin axial bewegbaren Schieber besteht. Der Applikator ist an seinem Einführende mit einer Kappe aus einer Kunststoffolie verschlossen. Über dem Einführende des Applikators ist eine Hülse aus der Kunststoffolie angeordnet, die über das Einführende des Applikators vorsteht. Das offene Ende der Hülse ist unter Bildung der erwähnten Kappe zugefaltet. Beim Gebrauch des Applikators wird durch axiales Drücken auf den Schieber der Tampon von innen gegen das zugefaltete Ende der Kappe gepreßt, das sich dabei öffnet und den Weg für das Ausschieben des Tampons freigibt.

Tampons dieser Art vergrößern durch Aufnahme von Feuchtigkeit oder Flüssigkeit ihr Volumen. Geschieht dies, solange sich der Tampon noch im Applikator befindet, z. B. bei Lagerung in feuchter Umgebung, kann sich der Tampon in der Applikatorhülse durch Volumenvergrößerung verklemmen und ein Ausstoßen aus dem Applikator erheblich erschweren. Es muß deshalb der Tampon durch geeignete Mittel an einer wesentlichen Volumenvergrößerung gehindert werden, was z. B. durch Ausschluß von Wasserdampf möglich ist.

Die Kappe des bekannten Tamponapplikators könnte zwar, wenn sie aus einem geeigneten Material besteht, das Eindringen von Wasserdampf durch das Einführende des Applikators in den Tampon verhindern. Jedoch kann das rückwärtige Tamponende den Wasserdampf noch durch den hohlen Schieber und/oder durch den Spalt zwischen Schieber und Applikatorhülse aufnehmen. Deshalb sind weitere Maßnahmen erforderlich, um eine Volumenvergrößerung des Tampons in dem bekannten Applikator durch Aufnahme von Wasserdampf zu vermeiden.

Der Erfindung liegt die Aufgabe zugrunde, eine Tamponpackung der eingangs genannten Art so auszubilden, daß die Tamponpackung auch nach längerer Lagerzeit, insbesondere in einer relativ feuchten Umgebung, ihre Funktion erfüllt, also eine durch Wasserdampfaufnahme verursachte Volumenvergrößerung des Tampons, die sein Ausstoßen aus dem Applikator erschweren würde, verhindert wird. Gleichzeitig soll die pro Tamponpackung erforderliche und bei diesem Massenartikel ins Gewicht fallende Menge an wertvollem, wasserdampfdichtem Verpackungsmaterial verringert werden.

Diese Aufgabe wird gemäß der Erfindung dadurch gelöst, daß die Verpackungsfolie im wesentlichen die gesamte Oberfläche des Tampons umgibt, im wesentlichen wasserdampfdicht ausgebildet ist und an der dem Tampon zugekehrten Innenseite mit einer einen hohen Gleitwert aufweisenden Beschichtung versehen ist.

Dadurch wird im Normalfall verhindert, daß der Tampon während seiner Lagerung größere Mengen an Wasserdampf aufnimmt und somit sein Volumen wesentlich erhöht. Wenn trotzdem in Ausnahmefällen der verpackte Tampon aus irgendeinem Grund zu expandieren versucht, wird seine Volumenvergrößerung durch die Verpackungsfolie, die an der Tamponoberfläche straff anliegt, praktisch allseitig begrenzt. Außerdem gewährleistet in diesen Fällen die Beschichtung mit einem hohen Gleitwert an der Innenseite der Verpackungsfolie, daß Gleitwert an der Innenseite der Verpackungsfolie, daß der Tampon noch relativ leicht aus dieser Folie und aus der Applikatorhülse herausgedrückt werden kann.

Das Umhüllen nur der Tampons mit einer wasserdampfdichten Verpackungsfolie ermöglicht ein längeres Zwischenlagern der verpackten Tampons, bevor sie mit den Applikatoren konfektioniert werden. Während des Zwischenlagerns behalten die Tampons im wesentlichen das ihnen bei der Herstellung verliehene Volumen und können deshalb gewünschtenfalls zu einem späteren Zeitpunkt in die Applikatoren eingebracht werden.

Durch die Tatsache, daß nur der eigentliche Tampon und nicht mehr die gesamte Anordnung aus Applikator und Tampon von einer relativ hochwertigen Verpackungsfolie umgeben ist, wird eine große Menge dieses Materials eingespart. Für die Umhüllung der gesamten Anordnung aus Applikator und Tampon genügt dann ein einfacheres, z. B. mechanisch weniger widerstandsfähiges, und daher kostengünstigeres Verpackungsmaterial. Dabei ist auch möglich, eine Mehrzahl der Tamponpackungen in einer gemeinsamen Verpackung zusammenzufassen, wodurch weiteres Verpackungsmaterial eingespart wird, ohne auf eine Verpackung eines jeden einzelnen Tampons verzichten zu müssen.

Verschiedene bevorzugte Ausführungsformen der erfindungsgemäßen Tamponpackung sind in den Patentansprüchen 2 bis 11 angegeben.

Gemäß Anspruch 12 ist es zweckmäßig, wenn das vordere Ende des Schiebers zylindrisch ist und einen kleineren Außendurchmesser als der Tampon aufweist. Dadurch wird zwischen dem vorderen Ende des Schiebers und der Applikatorhülse ein Ringraum geschaffen, der das Umstülpen der Verpackungsfolie am hinteren Tamponende beim Ausstoßen des Tampons aus der Applikatorhülse erleichtert. Der kleinere Außendurchmesser des vorderen Schieberendes führt auch zu einer Einsparung an Schiebermaterial.

Schließlich ist es vorteilhaft, wenn der Rand der rückwärtigen Öffnung der Applikatorhülse nach innen umgebördelt ist und der Schieber auf dem in der Applikatorhülse befindlichen Längenabschnitt einen Ringwulst aufweist. Das rückwärtige Ende der Applikatorhülse dient somit als hinterer Anschlag für den Ringwulst des Schiebers, so daß ein unbeabsichtigtes Herausgleiten des Schiebers aus der Applikatorhülse vermieden wird.

Nachfolgend ist die Erfindung anhand von in der Zeichnung schematisch dargestellten Ausführungsbeispielen erläutert. Es zeigen :

Figur 1 bis 8 axiale Längsschnitte durch verschiedene Ausführungsformen der Tamponpackung und

Figur 9 einen Querschnitt durch die Tamponpackung gemäß Fig. 8 nach Schnittlinie A-A.

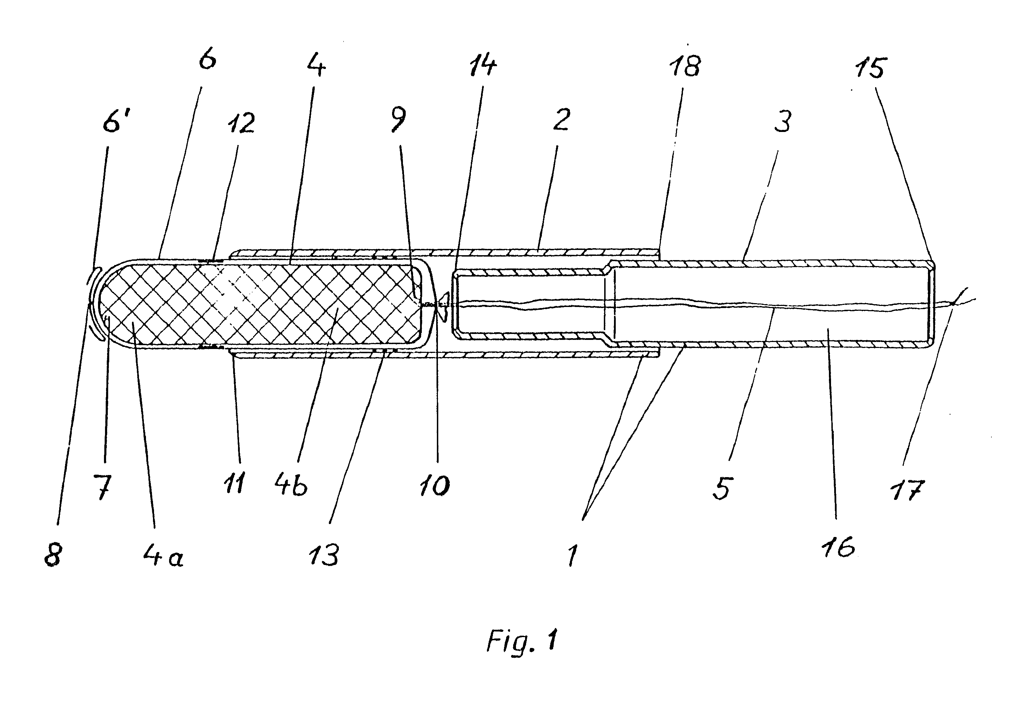

Gemäß den Fig. 1 bis 8 besteht die Tamponpackung im wesentlichen aus einem Tamponapplikator 1, der eine Applikatorhülse 2 und einen darin teleskopartig axial bewegbar gelagerten Schieber 3 aufweist, sowie aus einem in der Applikatorhülse 2 angeordneten Tampon 4 mit einem vorderen Tamponabschnitt 4a und einem rückwärtigen Tamponabschnitt 4b und mit einem Rückholband 5.

Gemäß Fig. 1 umgibt eine wasserdampfdichte Verpackungsfolie 6, z. B. aus Cellulosehydrat oder regenerierter Cellulose, im wesentlichen die gesamte Oberfläche des Tampons 4. Die Verpackungsfolie 6 ist auf der dem Tampon 4 zugekehrten Seite mit einer in der Zeichnung nicht dargestellten, einen hohen Gleitwert aufweisenden Beschichtung versehen. Diese besteht z. B. aus einer Polymerlackierung. Am Einführende 7 des Tampons 4 weist die Verpackungsfolie 6 einen üblichen vorderen Verschluß 8 auf, der vorzugsweise durch Raffsiegelung gebildet worden ist. In der Zeichnung ist am Verschluß 8 das vordere Ende 6' der Verpackungsfolie 8 das vordere Ende 6' der Verpackungsfolie 6 zur Verdeutlichung nicht eng anliegend dargestellt.

Am rückwärtigen Ende 9 des Tampons 4 ist ein rückwärtiger Verschluß 10 der Verpackungsfolie 6 vorgesehen.

Der Tampon 4 ragt mit dem vorderen Tamponabschnitt 4a über das Einführende 11 der Applikatorhülse 2 hinaus, und die Verpackungsfolie 6 ist an diesem Abschnitt 4a mit einem Aufreißband 12 in Umfangsrichtung des Tampons 4 versehen. Dadurch kann in einfacher Weise die Verpackungsfolie 6 vom Einführende 7 des Tampons 4 vor dessen Gebrauch abgetrennt werden.

Die Verpackungsfolie 6 ist mit demjenigen Abschnitt, welcher den innerhalb der Applikatorhülse 2 befindlichen Abschnitt 4b des Tampons 4 zylindrisch umgibt, an der Innenseite der Applikatorhülse 2 mittels Verbindungsstellen 13 befestigt und dadurch axial festgelegt.

Die Verbindungsstellen 13 können z. B. durch Einsatz eines Klebstoffes oder durch Heißsiegeln gebildet sein. Die Fläche einer solchen Verbindungsstelle 13 kann z. B. im wesentlichen punktförmig, streifenförmig oder dem Innenumfang der Applikatorhülse 2 folgend ringförmig sein. Soweit sich die Applikatorhülse 2 und die Verpackungsfolie 6 berühren, können beide auch vollflächig miteinander verbunden sein, wobei die Verbindungsstelle 13 dann eine zylinderförmige Fläche darstellt.

In der Applikatorhülse 2, die über ihre Gesamtlänge einen im wesentlichen konstanten Außendurchmesser und einen im wesentlichen konstanten Innendurchmesser aufweist, ist das vordere Ende 14 des Schiebers 3 angeordnet. Dieses Ende 14 ist zylindrisch gestaltet und weist einen kleineren Außendurchmesser als der Tampon 4 auf. Dadurch wird beim Ausstoßen des Tampons 4 aus der Applikatorhülse 2 das Umstülpen der in der Applikatorhülse 2 verbleibenden Verpackungsfolie 6 erleichtert.

Der restliche, bis zum rückwärtigen Ende 15 des Schiebers 3 sich erstreckende Schieberabschnitt hat gegenüber dem vorderen Ende 14 des Schiebers 3 einen größeren Außendurchmesser, der zur Führung des Schiebers 3 in der Applikatorhülse 2 nur geringfügig kleiner ist als der Innendurchmesser der Applikatorhülse 2.

Das Rückholband 5 erstreckt sich vom rückwärtigen Ende 9 des Tampons 4 durch den rückwärtigen Verschluß 10 der Verpackungsfolie 6 und einen Hohlraum 16 im Schieber 3 hindurch bis über dessen rückwärtiges Ende 15 hinaus, wo das Ende 17 des Rückholbandes 5 erfaßt werden kann.

Zur Benutzung der Tamponpackung wird zunächst das Aufreißband 12 abgelöst und das Einführende 7 des Tampons 4 von der Verpackungsfolie 6 befreit. Dann wird der Tamponapplikator 1 mit dem Tampon 4 in die Körperhöhle eingeführt. Durch Festhalten der Applikatorhülse 2 und gleichzeitiges axiales Drücken in der Einführrichtung auf den Schieber 3 wird der Tampon 4 aus der Applikatorhülse 2 und dem restlichen Teil der Verpackungsfolie 6 ausgestoßen. Beim Hinausgleiten des Tampons 4 aus der Applikatorhülse 2 wird die Verpackungsfolie 6 am rückwärtigen Ende 9 des Tampons 4 umgestülpt. Nachdem der Tampon 4 aus der Applikatorhülse 2 ausgestoßen und in der Körperhöhle richtig positioniert worden ist, wird der Tamponapplikator 1 mit der daran gehaltenen Verpackungsfolie 6 aus der Körperhöhle zurückgezogen.

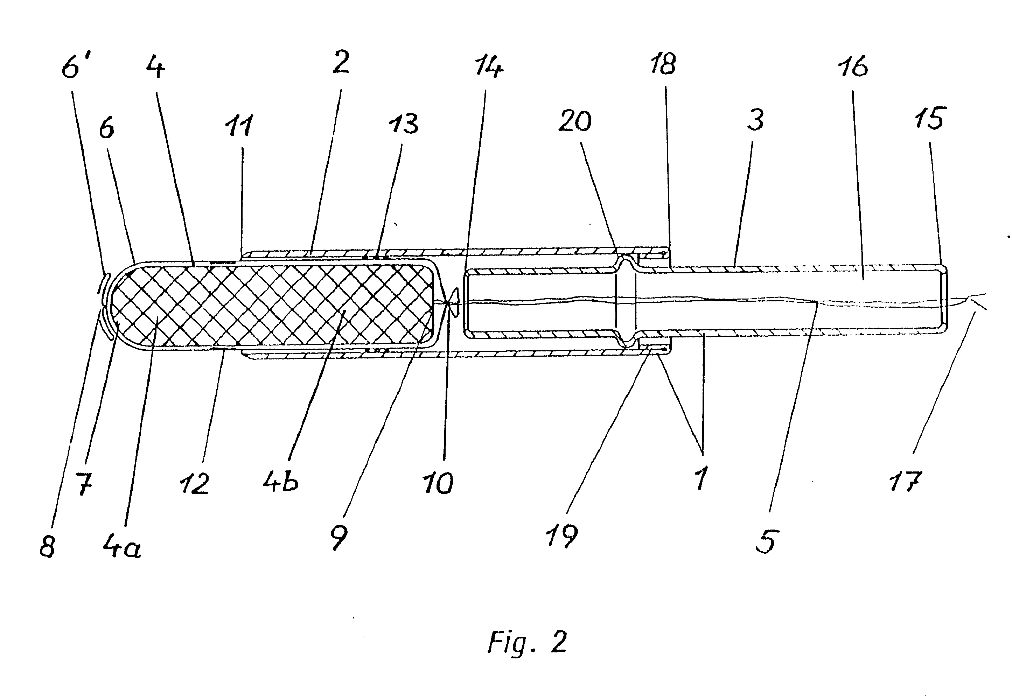

Fig. 2 unterscheidet sich von Fig. 1 dadurch, daß der Außendurchmessers des Schiebers 3 über dessen gesamte Länge im wesentlichen gleich bemessen ist und dem kleineren Außendurchmesser am vorderen Ende 14 des Schiebers 3 gemäß Fig. 1 entspricht. Jedoch ist bei der Ausführungsform gemäß Fig. 2 an der rückwärtigen Öffnung 18 der Applikatorhülse 2 deren Rand 19 nach innen umgebördelt, und der Schieber 3 weist auf dem in der Applikatorhülse 2 befindlichen Längenabschnitt einen Ringwulst 20 auf.

Der umgebördelte Rand 19 der Applikatorhülse 2 stellt einen Anschlag für den Ringwulst 20 des Schiebers 3 dar, wodurch ein unbeabsichtigtes Herausgleiten des Schiebers 3 aus der Applikatorhülse 2 vermieden wird.

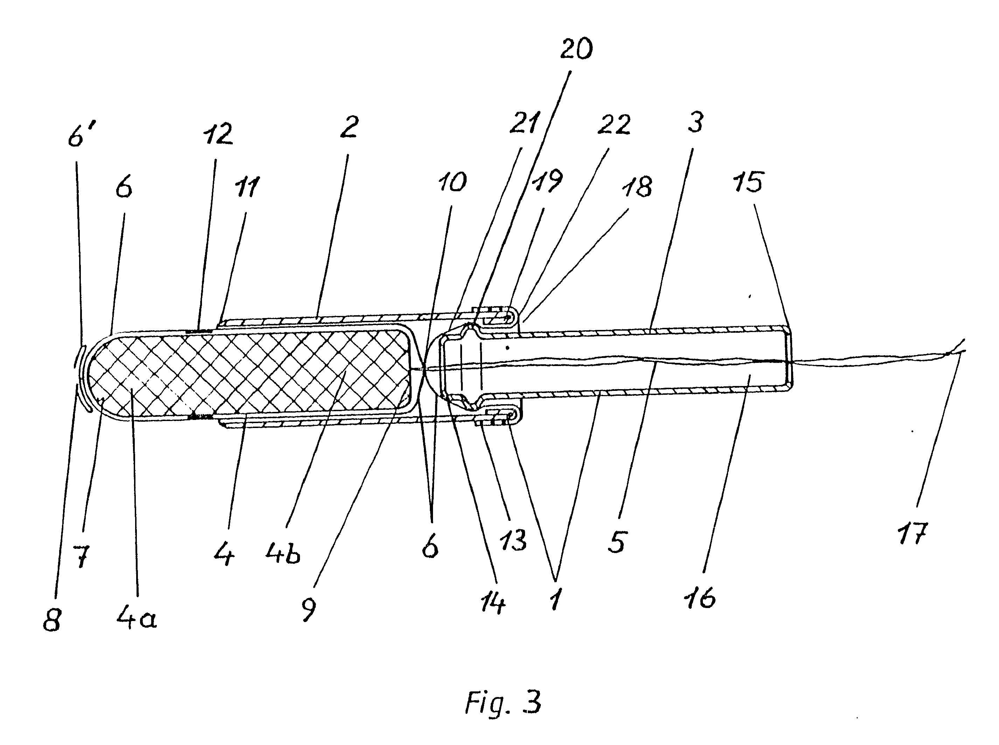

Gemäß Fig. 3 ist die Verpackungsfolie 6 zwar, wie bei den Ausführungsformen gemäß Fig. 1 und 2, am rückwärtigen Ende 9 des Tampons 4 verschlossen. Jedoch erstreckt sich die Verpackungsfolie 6 von ihrem rückwärtigen Verschluß 10 in Form einer sackartigen Verlängerung 21 durch die rückwärtige Öffnung 18 der Applikatorhülse 2 hindurch. Der Randbereich 22 der Verlängerung 21 ist um den rückwärtigen Rand 19 der Applikatorhülse 2 nach außen umgelegt und dort an den Verbindungsstellen 13 z. B. durch Ankleben oder Ansiegeln an der Applikatorhülse 2 befestigt. Der im Innern der Applikatorhülse 2 befindliche Abschnitt des Schiebers 3 wird von der sackartigen Verlängerung 21 der Verpackungsfolie 6 umgeben. Außerdem ist dieser Abschnitt des Schiebers 3 im Vergleich zu Fig. 2 wesentlich kürzer ausgebildet, wordurch Material für den Schieber 3 und die Applikatorhülse 2 gespart wird. Diese Verkürzung ist auch bei den anderen in der Zeichnung dargestellten Ausführungsformen der Tamponpackungen möglich.

Bei der Benutzung der Tamponpackung gemäß Fig. 3 wird nach dem Entfernen der Verpackungsfolie 6 vom Einführende 7 des Tampons 4 der Schieber 3 in Richtung auf den Tampon 4 gedrückt. Dabei öffnet sich der rückwärtige Verschluß 10 der Verpackungsfolie 6, und das vordere Ende 14 des Schiebers 3 stößt an das rückwärtige Ende 9 des Tampons 4, der dann wie üblich aus der Applikatorhülse 2 herausgeschoben wird.

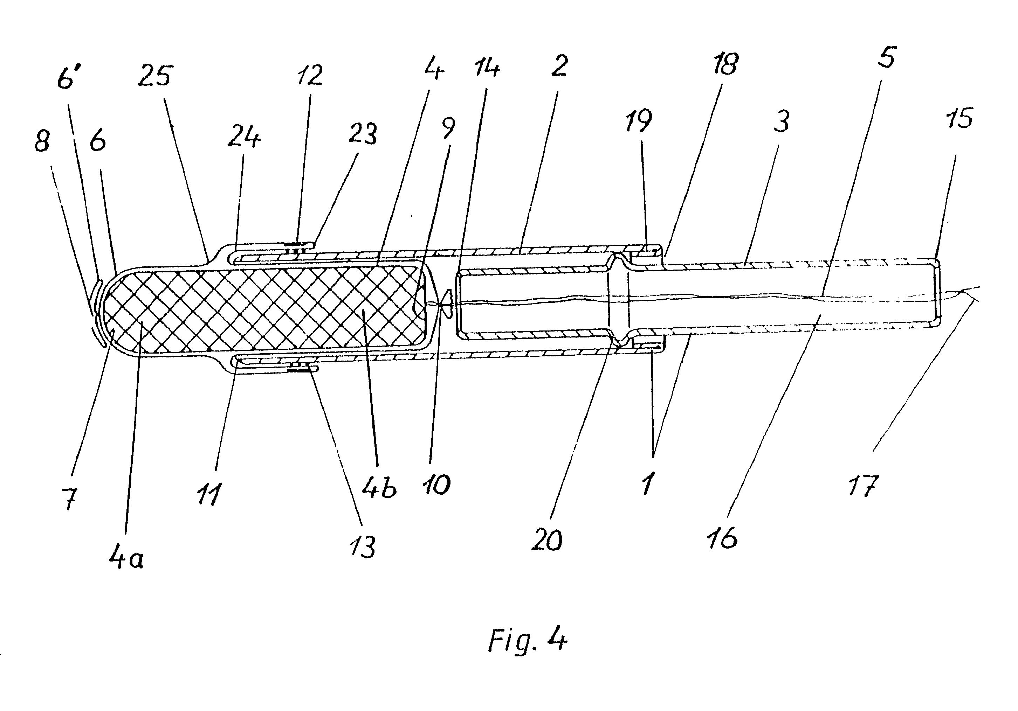

Gemäß Fig. 4 ist die Verpackungsfolie 6 im Bereich des Einführendes 11 der Applikatorhülse 2 an dieser befestigt. Die Verpackungsfolie 6 weist dort, wo sie den zylindrischen Abschnitt des Tampons 4 umgibt, eine doppelwandige Umfangslasche 23 mit einer inneren Laschenwand 24 und einer äußeren Laschenwand 25 auf. Die Umfangslasche 23 umgibt taschenartig das Einführende 11 der Applikatorhülse 2. Die innere Laschenwand 24 ist an Verbindungsstellen 13 auf der Außenseite der Applikatorhülse 2 befestigt und geht in denjenigen Abschnitt der Verpackungsfolie 6 über, welcher sich ins Innere der Applikatorhülse 2 und zum rückwärtigen Ende 9 des Tampons 4 erstreckt. Die äußere Laschenwand 25 der Verpackungsfolie 6 geht in deren vorderen Abschnitt über, welcher das Einführende 7 des Tampons 4 umhüllt. Das Aufreißband 12 der Verpackungsfolie 6 ist auf der äußeren Laschenwand 25 angeordnet.

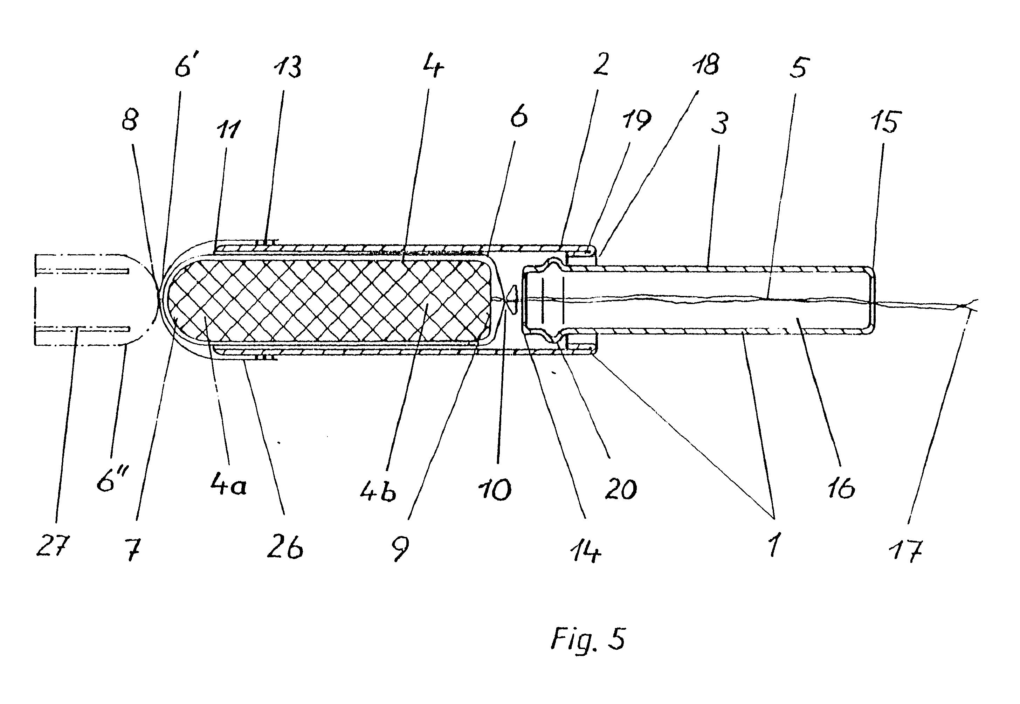

Gemäß Fig. 5 ist das vordere Ende 6' der Verpackungsfolie 6 am Einführende 7 des Tampons 4 so lang bemessen, daß dieses Ende 6' umgestülpt und unter Bildung einer Verschlußkappe 26 über das Einführende 11 der Applikatorhülse 2 gelegt und daran mittels Verbindungsstellen 13 befestigt werden kann. Die Verpackungsfolie 6 und die Verschlußkappe 26 bestehen also aus demselben Material.

Zur Verdeutlichung der Herstellung dieser Ausführungsform ist in Fig. 5 zusätzlich vor der Verschlußkappe 26 mit gestrichelten Linien das vordere Ende 6' der Verpackungsfolie 6 in Form eines Zylinders 6'' mit abgerundetem Boden dargestellt. Diese Form des Zylinders 6'' nimmt das Ende 6' der Verpackungsfolie 6 an, bevor es unter Bildung der Verschlußkappe 26 über das Einführende 11 der Applikatorhülse 2 gestülpt wird. Zur Erleichterung des Umstülpens weist der Zylinder 6'' achsparallele Schlitze 27 auf, von denen zwei in Fig. 5 gezeigt sind.

Zum Ausstoßen des Tampons 4 aus der Applikatorhülse 2 wird der vordere Verschluß 8 der Verpackungsfolie 6 durch den axialen Druck des Tampons 4 geöffnet. Deshalb ist bei dieser Ausführungsform der Tamponpackung das Aufreißband 12 überflüssig.

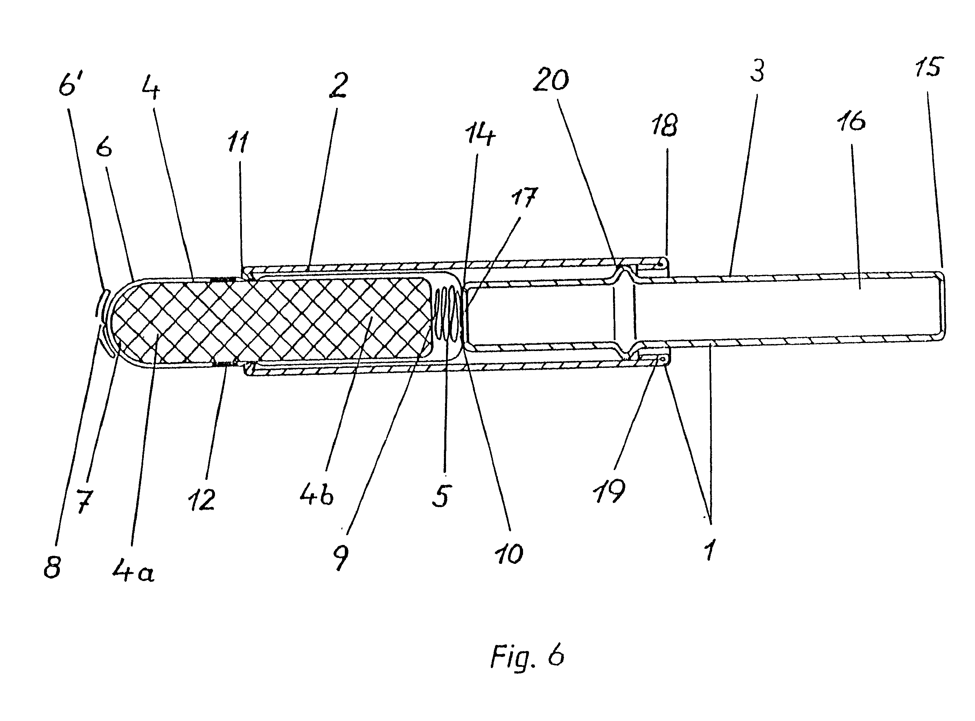

Gemäß Fig. 6 ist die Verpackungsfolie 6 zwischen dem Tampon 4 und der im Durchmesser verringerten Innenseite des Einführendes 11 der Applikatorhülse 2 nur radial eingeklemmt. Das Einführende 11 ist etwas nach innen gezogen und stützt sich gegen den Umfang der Verpackungsfolie 6 und den darunterliegenden Tampon 4 ab. Beim Ausschieben des Tampons 4 aus der Applikatorhülse 2 staut sich die Verpackungsfolie 6 in der Applikatorhülse 2.

Aus Fig. 6 ist auch ersichtlich, daß das Rückholband 5 an das rückwärtige Ende 9 des Tampons 4 angelegt und zusammen mit dem Tampon 4 von der Verpackungsfolie 6 umgeben ist. Damit in diesem Fall das Rückholband 5 nach dem Einführen des Tampons 4 in eine Körperhöhle gestreckt ist und aus der Körperhöhle herausragt, ist das rückwärtige Ende 17 des Rückholbandes 5 an dem rückwärtigen Verschluß 10 der Verpackungsfolie 6 befestigt. Wenn nach dem Einführen des Tampons 4 in eine Körperhöhle der Tamponapplikator 1 und damit die zwischen dem Einführende 11 der Applikatorhülse 2 und dem Schieber 3 eingeklemmte Verpackungsfolie 6 zurückgezogen wird, wird das Rückholband 5 in der Zugrichtung gestreckt, so daß sein rückwärtiges Ende 17 schließlich außerhalb der Körperhöhle angeordnet ist. Jedoch kann bei der Ausführungsform der Tamponpackung gemäß Fig. 6 das Rückholband 5 auch gestreckt angeordnet werden, wie es für die übrigen Ausführungsformen gezeigt ist.

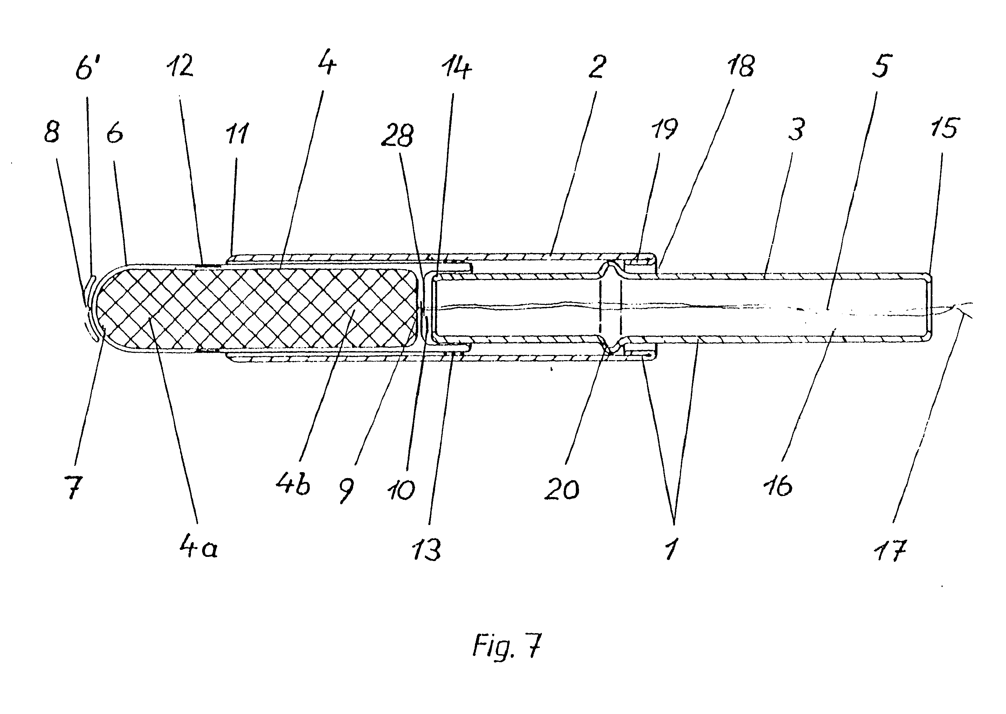

Gemäß Fig. 7 weist die Verpackungsfolie 6 am rückwärtigen Ende 9 des Tampons 4 eine zur Applikatorhülse 2 koaxiale Einstülpung 28 auf, in die das vordere Ende 14 des Schiebers 3 eingreift. Dies hat den Vorteil, daß die Verpackungsfolie 6 im Bereich der Einstülpung 28 leicht mittels eines in der Zeichnung nicht dargestellten spreizbaren Siegeldornes von innen an die Applikatorhülse 2 angesiegelt werden kann. Auf diese Weise wird während des Siegelns ein schnellerer Wärmeübergang auf die Verpackungsfolie 6 erreicht als im Falle einer Wärmezufuhr über die Außenseite der Applikatorhülse 2.

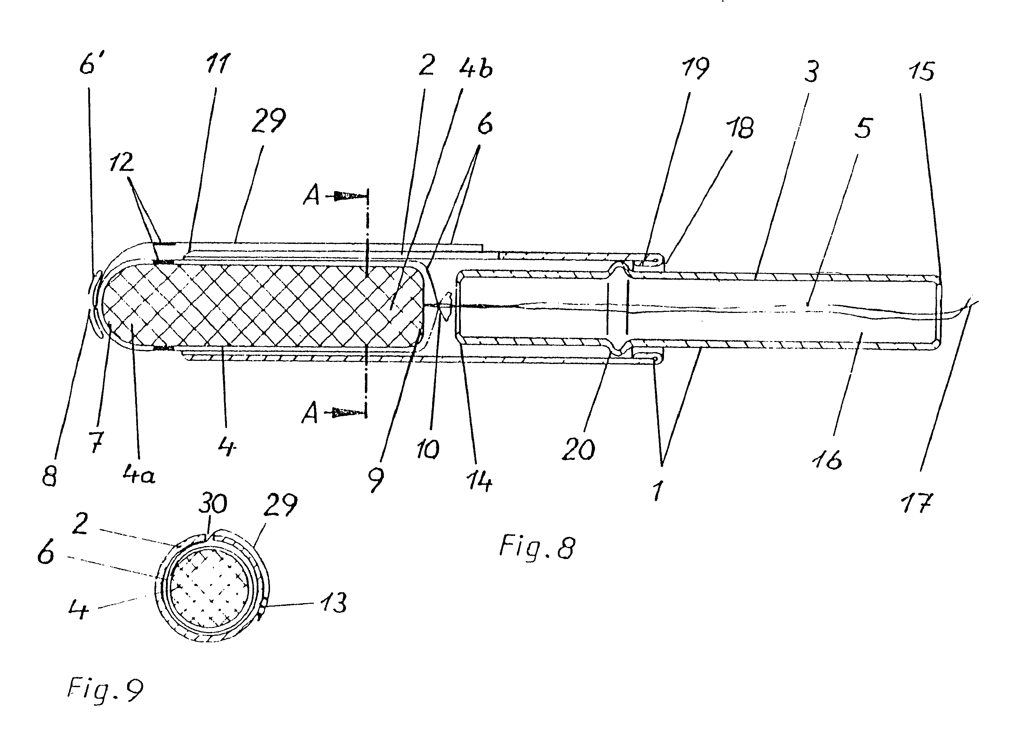

Gemäß Fig. 8 ist die Verpackungsfolie 6 an der Außenseite der Applikatorhülse 2 mittels einer einen Bestandteil der Verpackungsfolie 6 bildenden Befestigungslasche 29 vorgesehen, die durch einen axialen Schlitz 30 in der Applikatorhülse 2 aus dieser herausgeführt ist. Der Schlitz 30 erstreckt sich bis zum Einführende 11 der Applikatorhülse 2. Die Befestigungslasche 29 ist auf der Außenseite der Applikatorhülse 2 an Verbindungsstellen 13 z. B. durch Verkleben oder Versiegeln befestigt.

In der Fig. 9 ist ein Querschnitt durch die Tamponpackung gemäß Fig. 8 nach Schnittlinie A-A dargestellt und zeigt, daß die Verpackungsfolie 6 den Tampon 4 umgibt und sich dabei im Bereich des axialen Schlitzes 30 der Applikatorhülse 2 in einem kurzen Abschnitt überlappt.

Die vorstehend beschriebenen Tamponpackungen eignen sich für alle üblichen Hygienetampons.

Die Applikatorhülse 2 und der Schieber 3 können aus üblichen Materialien, z. B. aus Kunststoff oder Pappröhrchen, bestehen.

**Patentansprüche**

1. Tamponpackung, insbesondere für die Frauenhygiene, bestehend aus einem Tamponapplikator (1), der eine Applikatorhülse (2) und einen darin teleskopartig axial bewegbar gelagerten Schieber (3) aufweist, sowie aus einem in der Applikatorhülse (2) angeordneten Tampon (4) mit Rückholband (5), der mittels des Schiebers (3) aus der Applikatorhülse (2) ausstoßbar ist, wobei eine flexible Verpackungsfolie (6) im Bereich des Einführendes (7) des Tampons (4) angeordnet und an der Applikatorhülse (2) gehalten ist, dadurch gekennzeichnet, daß die Verpackungsfolie (6) im wesentlichen die gesamte Oberfläche des Tampons (4) umgibt, im wesentlichen wasserdampfdicht ausgebildet ist und an der dem Tampon (4) zugekehrten Innenseite mit einer einen hohen Gleitwert aufweisenden Beschichtung versehen ist.

2. Tamponpackung nach Anspruch 1, dadurch gekennzeichnet, daß der Tampon (4) mit einem axialen Längenabschnitt (4a) über das Einführende (11) der Applikatorhülse (2) hinausragt und die Verpackungsfolie (6) in diesem Längenabschnitt (4a) ein Aufreißband (12) in Umfangsrichtung des Tampons (4) aufweist. (Fig. 1).

3. Tamponpackung nach den Ansprüchen 1 oder 2, dadurch gekennzeichnet, daß die Verpackungsfolie (6) an der Innenseite der Applikatorhülse (2) gehalten ist. (Fig. 1, 2, 3, 6, 7).

4. Tamponpackung nach Anspruch 3, dadurch gekennzeichnet, daß die Verpackungsfolie (6) an der Innenseite der Applikatorhülse (2) befestigt ist. (Fig. 1, 2, 3, 7).

5. Tamponpackung nach Anspruch 3, dadurch gekennzeichnet, daß die Verpackungsfolie (6) zwischen dem Tampon (4) und dem Einführende (11) der Applikatorhülse (2) radial eingeklemmt und das rückwärtige Ende (17) des Rückholbandes (5) am rückwärtigen Verschluß (10) der Verpackungsfolie (6) befestigt sind. (Fig. 6).

6. Tamponpackung nach einem der Ansprüche 1 bis 3, dadurch gekennzeichnet, daß die Verpackungsfolie (6) an der Außenseite der Applikatorhülse (2) befestigt ist. (Fig. 4, 5, 8).

7. Tamponpackung nach Anspruch 6, dadurch gekennzeichnet, daß sich die Verpackungsfolie (6) durch die rückwärtige Öffnung (18) der Applikatorhülse (2) hindurch erstreckt und im Bereich dieser Öffnung (18) an der Applikatorhülse (2) befestigt ist. (Fig. 3).

8. Tamponpackung nach Anspruch 6, dadurch gekennzeichnet, daß die Verpackungsfolie (6) auch das Einführende (11) der Applikatorhülse (2) umgibt. (Fig. 4, 5).

9. Tamponpackung nach Anspruch 8, dadurch gekennzeichnet, daß das Aufreißband (12) der Verpackungsfolie (6) auf einer das Einführende (11) der Applikatorhülse (2) taschenartig umgebenden Umfangslasche (23) der Verpackungsfolie (6) vorgesehen ist. (Fig. 4).

10. Tamponpackung nach Anspruch 6, dadurch gekennzeichnet, daß die Befestigung der Verpackungsfolie (6) mittels einer an ihr ausgebildeten Befestigungslasche (29) vorgesehen ist, die durch einen axialen Schlitz (30) in der Applikatorhülse (2) aus dieser herausgeführt ist. (Fig. 8, 9).

11. Tamponpackung nach einem der Ansprüche 1 bis 3, dadurch gekennzeichnet, daß am rückwärtigen Ende (9) des Tampons (4) die Verpackungsfolie (6) eine zur Applikatorhülse (2) koaxiale Einstülpung (28) aufweist, in die das vordere Ende (14) des Schiebers (3) eingreift. (Fig. 7).

12. Tamponpackung nach einem der Ansprüche 1 bis 11, dadurch gekennzeichnet, daß das vordere Ende (14) des Schiebers (3) zylindrisch ist und einen kleineren Außendurchmesser als der Tampon (4) aufweist. (Fig. 1 bis 8).

13. Tamponpackung nach einem der Ansprüche 1 bis 12, dadurch gekennzeichnet, daß an der rückwärtigen Öffnung (18) der Applikatorhülse (2) deren Rand (19) nach innen umgebördelt ist und der Schieber (3) auf dem in der Applikatorhülse (2) befindlichen Längenabschnitt einen Ringwulst (20) aufweist. (Fig. 2 bis 8).

**Claims**

1. A tampon pack, in particular for female hygiene, consisting of a tampon applicator (1) having an applicator shell (2) and a plunger (3) telescopically mounted therein to be axially movable, and of a tampon (4) which is located in the applicator shell (2), has a withdrawal cord (5) and can be pushed out of the applicator shell (2) by means of the plunger (3), a flexible packaging film (6) being provided in the region of the introduction end (7) of the tampon (4) and being held on the applicator shell (2), wherein the packaging film (6) surrounds substantially the entire surface of the tampon (4), is made to be substantially impermeable to water-vapour and is provided, on the inside facing the tampon (4), with a coating having a high sliding value.

2. A tampon pack as claimed in claim 1, wherein an axial longitudinal portion (4a) of the tampon (4) projects beyond the introduction end (11) of the applicator shell (2) and the packaging film (6) has, in this longitudinal portion (4a), a tear-off strip (12) in the peripheral direction of the tampon (4). (Fig. 1).

3. A tampon pack as claimed in claim 1 or 2, wherein the packaging film (6) is held on the inside of the applicator shell (2). (Figs. 1, 2, 3, 6 and 7).

4. A tampon pack as claimed in claim 3, wherein the packaging film (6) is fixed to the inside of the applicator shell (2). (Figs. 1, 2, 3 and 7).

5. A tampon pack as claimed in claim 3, wherein the packaging film (6) is radially clamped in between the tampon (4) and the introduction end (11) of the applicator shell (2) and the rear end (17) of the withdrawal cord (5) is fixed to the rear closure (10) of the packaging film (6). (Fig. 6).

6. A tampon pack as claimed in any of claims 1 to 3, wherein the packaging film (6) is fixed to the outside of the applicator shell (2). (Figs. 4, 5 and 8).

7. A tampon pack as claimed in claim 6, wherein the packaging film (6) extends through the rear opening (18) of the applicator shell (2) and is fixed to the applicator shell (2) in the region of this opening (18). (Fig. 3).

8. A tampon pack as claimed in claim 6, wherein the packaging film (6) also surrounds the introduction end (11) of the applicator shell (2). (Figs. 4 and 5).

9. A tampon pack as claimed in claim 8, wherein the tear-off strip (12) of the packaging film (6) is provided on a peripheral strap (23) of the packaging film (6), which strap surrounds the introduction end (11) of the applicator shell (2) like a pocket. (Fig. 4).

10. A tampon pack as claimed in claim 6, wherein the fixing of the packaging film (6) is provided by means of a fixing strap (29) which is formed on the packaging film and is led out from the applicator shell (2) through an axial slot (30) in the latter. (Figs. 8 and 9).

11. A tampon pack as claimed in any of claims 1 to 3, wherein the packaging film (6) has, at the rear end (9) of the tampon (4), a tamp-in portion (28) which is coaxial to the applicator shell (2) and into which the front end (14) of the plunger (3) engages. (Fig. 7).

12. A tampon pack as claimed in any of claims 1 to 11, wherein the front end (14) of the plunger (3) is cylindrical and has an external diameter which is smaller than that of the tampon (4). (Figs. 1 to 8).

13. A tampon pack as claimed in any of claims 1 to 12, wherein the edge (19) of the applicator shell (2) is plunged inwards at the rear opening (18) thereof, and the plunger (3) has an annular bead (20) on the longitudinal portion located in the applicator shell (2). (Figs. 2 to 8).

**Revendications**

1. Garniture à tampon, notamment pour l'hygiène féminine, constituée par un applicateur (1) qui comporte un manchon (2) et un poussoir (3) monté de façon à pouvoir se déplacer axialement d'une manière télescopique à l'intérieur du manchon, ainsi qu'un tampon (4) disposé à l'intérieur du manchon (2) de l'applicateur et comportant une bande de rappel (5), et qui peut être repoussé hors du manchon (2) au moyen du poussoir (3), une feuille souple d'emballage (6) étant disposée au niveau de l'extrémité d'introduction (7) du tampon (4) et étant reliée au manchon (2) de l'applicateur, caractérisée en ce que la feuille d'emballage (6), entoure essentiellement l'ensemble de la surface du tampon (4), est réalisée de manière à être essentiellement étanche à la vapeur d'eau, et comporte, sur sa face intérieure tournée vers le tampon (4), un revêtement possédant un faible coefficient de frottement.

2. Garniture à tampon selon la revendication 1, caractérisée en ce que le tampon (4) fait saillie, par un élément de longueur axial (4a) hors de l'extrémité d'introduction (11) du manchon (2) de l'applicateur, et que la feuille d'emballage (6) possède, sur cet élément de longueur (4a), une bande d'arrachement (12) s'étendant suivant la direction circonférentielle du tampon (4).

3. Garniture à tampon selon la revendication 1 ou 2, caractérisée en ce que la feuille d'emballage (6) est maintenue sur la face intérieure du manchon (2) de l'applicateur (figures 1, 2, 3, 6, 7).

4. Garniture à tampon selon la revendication 3, caractérisée en ce que la feuille d'emballage (6) est fixée sur la face intérieure du manchon (2) de l'applicateur (figures 1, 2, 3, 7).

5. Garniture à tampon selon la revendication 3, caractérisée en ce que la feuille d'emballage (6) est bloquée radialement par serrage entre le tampon (4) et l'extrémité d'introduction (11) du manchon (2) de l'applicateur et que l'extrémité arrière (17) de la bande de rappel (5) est fixée à l'élément de fermeture arrière (10) de la feuille d'emballage (6) (figure 6).

6. Garniture à tampon selon l'une des revendications 1 à 3, caractérisée en ce que la feuille d'emballage (6) est fixée sur la face extérieure du manchon (2) de l'applicateur (figures 4, 5, 8).

7. Garniture à tampon selon la revendication 6, caractérisée en ce que la feuille d'emballage (6) s'étend à travers l'ouverture arrière (18) du manchon (2) de l'applicateur et est fixée à ce manchon (2), au voisinage de cette ouverture (18) (figure 3).

8. Garniture à tampon selon la revendication 6, caractérisée en ce que la feuille d'emballage (6) entoure également l'extrémité d'introduction (11) du manchon (2) de l'applicateur (figures 4, 5).

9. Garniture à tampon selon la revendication 8, caractérisée en ce que la bande d'arrachement (12) de la feuille d'emballage (6) est prévue sur une languette périphérique (23) de la feuille d'emballage (6), qui entoure à la manière d'une poche l'extrémité d'introduction (11) du manchon (2) de l'applicateur (figure 4).

10. Garniture à tampon selon la revendication 6, caractérisée en ce que la fixation de la feuille d'emballage (6) est réalisée au moyen d'une languette de fixation (29) formée dans cette feuille et qui ressort du manchon (2) de l'applicateur à travers une fente axiale (30) ménagée dans ce dernier (figures 8, 9).

11. Garniture à tampon selon l'une des revendications 1 à 3, caractérisée en ce qu'au niveau de l'extrémité arrière (9) du tampon (4), la feuille d'emballage (6) possède un renfoncement (28) coaxial au manchon (2) de l'applicateur et dans lequel s'engage l'extrémité avant (14) du poussoir (3) (figure 7).

12. Garniture à tampon selon l'une des revendications 1 à 11, caractérisée en ce que l'extrémité avant (14) du poussoir (3) est cylindrique et possède un diamètre extérieur inférieur à celui du tampon (4) (figures 1 à 8).

13. Garniture à tampon selon l'une des revendications 1 à 12, caractérisée en ce que le bord (19) du manchon (2) de l'applicateur est muni d'un rebord rentrant au niveau de l'ouverture arrière (18) du manchon et que le poussoir (3) comporte un bourrelet annulaire (20) au niveau de l'élément de longueur situé sur le manchon (2) de l'applicateur (figures 2 à 8).

6' 6 4 14 18 15

12 9 2 3

7 11 4b 10 1 5 17

8 4a 13 1 16

0 134 227

Fig. 1

*Fig. 2*

0 134 227

Fig. 3

0 134 227

Fig. 4

Fig. 5

0 134 227

Fig. 6

Fig. 7

0 134 227

Fig.8

Fig.9

0 134 227